# FASCICULE DE BREVET EUROPEEN

(11) **EP 1 842 528 B1**
(45) Date de publication et mention de la délivrance du brevet: **15.04.2015**
(21) Numéro de dépôt: 07103053.0
(22) Date de dépôt: 26.02.2007
(51) Int. Cl.: A61K 8/68, A61K 8/36, A61K 8/60, A61Q 5/00, A61Q 5/02, A61K 8/365, A61Q 1/10, A61Q 5/12

(54) **Composition cosmétique comprenant au moins un hydroxy acide, au moins un mono ou disaccharide et au moins un céramide et le procédé de fabrication**
Kosmetische Zusammensetzung, enthaltend mindestens eine Hydroxysäure, eine Mono- oder Disaccharide und mindestens ein Ceramide und deren Verfahren zur Herstellung
Cosmetic composition comprising at least one hydroxy acid, at least one mono- or disaccharide and at least one ceramide, and process for the preparation thereof

(30) Priorité: 04.04.2006 FR 0651187
(43) Date de publication de la demande: 10.10.2007
(73) Titulaire: L'Oréal, 75008 Paris (FR)
(72) Inventeur: Mellul, Myriam, 75014 Paris (FR); Sturla, Jean-Michel, 92100, Boulogne (FR)
(74) Mandataire: Le Blainvaux Bellegarde, Françoise

(56) Documents cités:
- WO-A-2004/054525
- WO-A-2004/054526
- WO-A-2004/073663
- DE-B- 1 220 969
- JP-A- 2004 250 372
- DATABASE WPI Week 2004 Derwent Publications Ltd., London, GB; AN 2004-184801 XP002407833 "First agent for wavy-hair correction, contains reducer, alkali chemicals, gluconic acid and trehalose." & JP 2004 026770 A (MIRUBON KK) 29 janvier 2004 (2004-01-29)
- DATABASE WPI Week 2003 Derwent Publications Ltd., London, GB; AN 2003-527917 XP002407834 "Rinse for hair comprises polysaccharide containing glucose, fucose, glucuronic acid and/or rhamnose as main components" & JP 2003 089624 A (HAKUTO KK) 28 mars 2003 (2003-03-28)
- DATABASE WPI Week 2003, 02 Février 2003 Derwent Publications Ltd., London, GB; AN 2004-630681 'Drink useful as refined sake and for use in skin external preparation e.g. cosmetics for preventing and improving rough skin, aging, wrinkles and dullness of skin, contains component effective in skin cosmetics and alcohol' & JP 2004 250372 A (FANKERU KK)

## Description

La présente invention a trait à une composition cosmétique pour le traitement des matières kératiniques, plus particulièrement les fibres kératiniques et notamment les cheveux, comprenant au moins un alpha hydroxy acide, au moins un mono ou disaccharide et au moins un composé de type céramide ainsi qu'au procédé de traitement non-thérapeutique à l'aide de cette composition.

On connaît déjà dans l'état de la technique des formulations capillaires permettant de traiter les cheveux abîmés par les intempéries ou les traitements capillaires physiques (brushing, peignage ...) ou chimiques (coloration, permanente...). On a déjà utilisé dans ce but les céramides ou les glycocéramides dans le but de protéger la fibre capillaire. L'application sur cheveux de ces dernières compositions ou des céramides seuls conduit cependant à des performances cosmétiques insuffisantes, tant sur cheveux mouillés que sur cheveux séchés.

Dans le domaine capillaire, des problèmes de casse des cheveux se posent fréquemment lorsque ceux-ci subissent un simple traitement mécanique comme le brossage, le peignage ou le lissage, ce type de traitement pouvant être effectué en association ou non avec des traitements cosmétiques concomitants ou consécutifs, comme un défrisage, un shampooing, une coloration ou une permanente. Par ailleurs, lors des traitements mécaniques tels que le brushing, les cheveux sont abîmés par la chaleur du séchoir et le passage de la brosse dans les cheveux pour mettre en forme la chevelure.

Les problèmes de casse sont par ailleurs particulièrement exacerbés sur certains cheveux très frisés où un phénomène de casse prématurées est observé (J. Soc. Cosmet. Chem., 36, janvier/février 1985, 39-52). Leur degré de frisure important s'oppose à un démêlage ou lissage aisé et requiert donc l'application de tensions mécaniques plus importantes. Pour des raisons évidentes, ces tensions affectent significativement la résistance mécanique des cheveux et génèrent généralement un taux important de casses.
De nombreux cheveux sont ainsi cassés lors d'un brushing. On recherche donc des compositions permettant de protéger les cheveux de cette casse lors de ces agressions.

Pour prévenir les phénomènes de casse du cheveu, il a déjà été proposé de traiter les cheveux, avec des composés de type céramide.
Les céramides ou leurs analogues sont connus pour protéger et/ou réparer la peau et/ou les fibres capillaires des agressions des divers agents et traitements cités ci-dessus. En particulier, ils ont un effet barrière qui limitent la fuite des protéines; ils renforcent également la cohésion cuticulaire.

Cependant, même si ces céramides s'avèrent efficaces, les propriétés de protection ou de réparation des compositions contenant de tels composés peuvent apparaître encore insuffisantes.

Notamment, il faut généralement effectuer plusieurs applications de la composition contenant des céramides.

Par ailleurs, il est également connu que les hydroxyacides ont un effet renforcateur de la fibre capillaire. Cependant, là encore il faut généralement effectuer plusieurs applications de la composition pour obtenir des effets suffisants concernant la casse des cheveux.

WO 2004/073663 et WO 2004/054526 traitent du problème de l'endommagement des cheveux provoqués par des agressions extérieures (UV) ou des traitements chimiques tels que l'éclaircissement, la permanente, ou encore le lavage fréquent des cheveux avec des compositions détergentes agressives. Afin de prévenir et réparer ces dommages occasionnés aux cheveux, WO 2004/073663 et WO 2004/054526 proposent d'utiliser des compositions comprenant l'association d'un disaccharide avec un diacide ou un diol spécifique.

De manière inattendue, les inventeurs ont constaté qu'une association particulière de céramide, d'alphahydroxyacide et de mono ou disaccharide s'avérait particulièrement avantageuse pour prévenir ce phénomène de casse de cheveux en réduisant le nombre d'applications nécessaires. Parallèlement on observe généralement une réduction de la quantité de cheveux recueillis après une application suivie d'un brushing.

Plus précisément, ils ont constaté qu'en utilisant des compositions contenant un hydroxyacide, un mono ou disaccharide et un composé de type céramide, notamment sur des cheveux très sensibilisés, on observait une amélioration de la résistance mécanique des cheveux, la quantité de cheveux recueillie après brushing était nettement diminuée.

En particulier, on obtenait un très bon effet de protection vis-à-vis de la casse des cheveux pendant un brushing, supérieur à celui obtenu soit avec l'hydroxyacide et le monosaccharide seuls, soit avec le composé de type céramide seul, ces composés utilisés à la quantités totales des composés de l'association. La demanderesse a constaté en particulier que l'association avait un effet de synergie qui n'est pas simplement l'addition des propriétés des trois composants.

Cette découverte est à la base de la présente invention.

L'invention a donc pour objet une composition cosmétique à application topique, destinée au traitement des matières kératiniques, notamment des fibres kératiniques en particulier les cheveux, caractérisée par le fait qu'elle comprend dans un milieu cosmétiquement acceptable au moins un monosaccharide et/ou un disaccharide, au moins un alphahydroxyacide et au moins un composé de type céramide.

L'invention a encore pour objet l'utilisation de la composition définie ci-dessus pour protéger les matières kératiniques, notamment des fibres kératiniques en particulier les cheveux, des agressions physiques ou chimiques et en particulier vis-à-vis des brushings.

L'invention a encore pour objet l'utilisation de la composition définie ci-dessus pour diminuer la masse de cheveux recueillie pendant les brushings.

Le terme "matière kératinique" selon l'invention englobe la peau, les ongles et les fibres kératiniques. On entend par "fibres kératiniques", les cheveux, les cils, les sourcils et les poils.

L'invention concerne également un procédé cosmétique de soin, de renforcement et/ou de réparation des matières kératiniques, caractérisé en ce que l'on applique sur lesdites matières kératiniques une composition telle que définie précédemment, suivi éventuellement d'un rinçage.

Par 'renforcement et/ou réparation des substrats kératiniques' selon l'invention, on entend une composition destinée à conserver et/ou restaurer et/ou améliorer les propriétés physiques et/ou mécaniques des substrats kératiniques, pouvant se manifester par exemple :
- soit par une meilleure élasticité et/ou une meilleure résistance à des forces mécaniques de traction qui leur sont appliquées, par exemple lors d'un peignage ou d'un brushing pour les fibres kératiniques, en particulier sur des cheveux africains ou tous cheveux fragilisés ou sensibilisés.

Lorsque la composition est appliquée sur les ongles, ils sont plus lisses, plus brillants.
Lorsque la composition est appliquée sur les cils, ils sont plus lisses, plus brillants, moins abîmés et moins cassants.

Selon la présente invention, on entend, par composé de type céramide, les céramides et/ou les glycocéramides et/ou les pseudocéramides et/ou les néocéramides, naturelles ou synthétiques.

Des composés de type céramides sont par exemple décrits dans les demandes de brevet DE4424530, DE4424533, DE4402929, DE4420736, WO95/23807, WO94/07844, EP-A-0646572, WO95/16665, FR-2 673 179, EP-A-0227994 et WO94/07844, WO94/24097, WO94/10131 dont les enseignements sont ici inclus à titre de référence.

Les composés de type céramide utilisables selon la présente invention répondent préférentiellement à la formule générale (I): 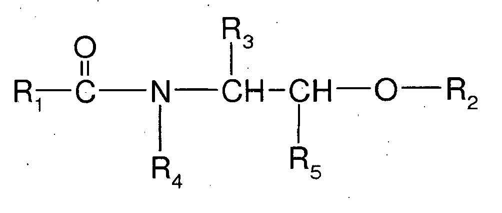 dans laquelle :
- R₁ désigne :

- soit un radical hydrocarboné, linéaire ou ramifié, saturé ou insaturé, en C₁-C₅₀, de préférence en C₅-C₅₀, ce radical pouvant être substitué par un ou plusieurs groupements hydroxyle éventuellement estérifié par un acide R₇COOH, R₇ étant un radical hydrocarboné, saturé ou insaturé, linéaire ou ramifié, éventuellement mono ou polyhydroxylé, en C₁-C₃₅ , le ou les hydroxyles du radical R₇ pouvant être estérifié par un acide gras saturé ou insaturé, linéaire ou ramifié, éventuellement mono
ou polyhydroxylé, en C₁-C₃₅;
- soit un radical R"-(NR-CO)-R', R désigne un atome d'hydrogène ou un radical hydrocarboné C₁-C₂₀ mono ou polyhydroxylé, préférentiellement monohydroxylé, R' et R" sont des radicaux hydrocarbonés dont la somme des atomes de carbone est comprise entre 9 et 30, R' étant un radical divalent.
- soit un radical R₈-O-CO-(CH2)ₚ, R₈ désigne un radical hydrocarboné en C₁-C₂₀, p est un entier variant de 1 à 12.
- R₂ est choisi parmi un atome d'hydrogène, un radical de type saccharidique, en particulier un radical (glycosyle)ₙ, (galactosyle)ₘ ou sulfogalactosyle, un résidu de sulfate ou de phosphate, un radical phosphoryléthylamine et un radical phosphoryléthylammonium, dans lesquels n est un entier variant de 1 à 4 et m est un entier variant de 1 à 8 ;
- R₃ désigne un atome d'hydrogène ou un radical hydrocarboné en C₁-C₃₃, saturé ou insaturé, hydroxylé ou non, le ou les hydroxyles pouvant être estérifiés par un acide minéral ou un acide R₇COOH, R₇ ayant les mêmes significations que ci-dessus, le ou les hydroxyles pouvant être éthérifiés par un radical (glycosyle)ₙ, (galactosyle)ₘ sulfogalactosyle, phosphoryléthylamine ou phosphoryléthylammonium, R₃ pouvant également être substitué par un ou plusieurs radicaux alkyle en C₁-C₁₄ ;
   de préférence, R₃ désigne un radical α-hydroxyalkyle en C₁₅-C₂₆, le groupement hydroxyle étant éventuellement estérifié par un α-hydroxyacide en C₁₆-C₃₀ ;
- R₄ désigne un atome d'hydrogène, un radical méthyle, éthyle, un radical hydrocarboné en C₃-C₅₀, saturé ou insaturé, linéaire ou ramifié, éventuellement hydroxylé ou un radical -CH₂-CHOH-CH₂-O-R₆ dans lequel R₆ désigne un radical hydrocarboné en C₁₀-C₂₆ ou un radical R₈-O-CO-(CH₂)ₚ, R₈ désigne un radical hydrocarboné en C₁-C₂₀, p est un entier variant de 1 à 12,
- R₅ désigne un atome d'hydrogène ou un radical hydrocarboné en C₁-C₃₀ saturé ou insaturé, linéaire ou ramifié, éventuellement mono ou polyhydroxylé, le ou les hydroxyles pouvant être éthérifiés par un radical (glycosyle)ₙ, (galactosyle)ₘ, sulfogalactosyle, phosphoryléthylamine ou phosphoryléthylammonium,
sous réserve que lorsque R₃ et R₅ désignent hydrogène ou lorsque R₃ désigne hydrogène et R₅ désigne méthyle alors R₄ ne désigne pas un atome d'hydrogène, un radical méthyle ou éthyle.

Parmi les composés de formule (I), on préfère les céramides et/ou glycocéramides dont la structure est décrite par DOWNING dans Journal of Lipid Research Vol. 35, 2060-2068, 1994, ou ceux décrits dans la demande de brevet français FR-2 673 179, dont les enseignements sont ici inclus à titre de référence.

Les composés de type céramide plus particulièrement préférés selon l'invention sont les composés de formule (I) pour lesquels R₁ désigne un alkyle saturé ou insaturé dérivé d'acides gras en C₁₄-C₂₂ éventuellement hydroxylé; R₂ désigne un atome d'hydrogène ; et R₃ désigne un radical linéaire en C₁₁₋₁₇ éventuellement hydroxylé et de préférence en C_{13-17.} R3 désigne de préférence un radical alpha hydroxycétyle, R₂, R₄ et R₅ désignent un atome d'hydrogène.

De tels composés sont par exemple :
- le 2-N-linoléoylamino-octadécane-1,3-diol,
- le 2-N-oléoylamino-octadécane-1,3-diol (N-oléoyldihydrosphingosine),
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol et en particulier la N-stéaroyl phytosphingosine,
- le 2-N-palmitoylamino-hexadécane-1,3-diol
ou les mélanges de ces composés.

On peut aussi utiliser des mélanges spécifiques tels que par exemple les mélanges de céramide(s) 2 et de céramide(s) 5 selon la classification de DOWNING.

On peut également utiliser les composés de formule (I) pour lesquels R₁ désigne un radical alkyle saturé ou insaturé dérivé d'acides gras en C₁₂-C₂₂ ; R₂ désigne un radical galactosyle ou sulfogalactosyle ; et R₃ désigne un radical hydrocarboné en C₁₂-C₂₂, saturé ou insaturé et de préférence un groupement -CH=CH-(CH₂)₁₂-CH₃.

A titre d'exemple, on peut citer le produit constitué d'un mélange de glycocéramides, vendu sous la dénomination commerciale GLYCOCER par la société WAITAKI INTERNATIONAL BIOSCIENCES.

On peut également utiliser les composés de formule (I) décrits dans les demandes de brevet EP-A-0227994, EP-A-0 647 617, EP-A-0 736 522 et WO 94/07844.

De tels composés sont par exemple le QUESTAMIDE H (bis-(N-hydroxyéthyl N-cétyl) malonamide) vendu par la société QUEST, le N-(2-hydroxyéthyl)-N-(3-cétyloxy-2-hydroxypropyl)amide d'acide cétylique .

On peut également utiliser le N-docosanoyl N-méthyl-D-glucamine décrit dans la demande de brevet WO94/24097.

La concentration en composés de type céramide peut varier de 0,0001% à 10% en poids environ par rapport au poids total de la composition, et de préférence de 0,001 à 5% environ et encore plus préférentiellement de 0,005 à 1 % en poids mieux de 0,0075 à 0,1 % en poids.

Avantageusement, le monosaccharide est choisi parmi le glucose, le fructose, le galactose, le mannose, le talose, le sorbose, le xylose, le lyxose, le fucose, le fucose, le arabinose, le rhamnose, le ribose, le ribulose, le xylulose, le sorbitol.
En ce qui concerne les disaccharides, ils sont notamment choisis parmi le maltose, le lactose, le cellobiose, le tréhalose, le saccharose.
Ces saccharides peuvent se présenter sous forme d'isomères L ou D.

De préférence, le saccharide est un monosaccharide.

Plus particulièrement, le monosaccharide est choisi parmi le glucose, le fructose, et leurs mélanges, plus particulièrement sous forme d'isomère D.
Plus particulièrement, le disaccharide est le saccharose.

La concentration en monosaccharide selon l'invention peut varier de 0,05% à 10% en poids environ par rapport au poids total de la composition, et de préférence de 0,1 à 5% environ et encore plus préférentiellement de 0,1 à 1 % en poids.

Par alpha hydroxy acide, on entend selon la présente invention un acide carboxylique ayant au moins une fonction hydroxy occupant une position alpha sur ledit acide (carbone adjacent à une fonction acide carboxylique). Cet acide peut se présenter dans la composition finale sous forme d'acide libre et/ou sous la forme de l'un de ses sels associés (sels avec une base organique ou un alcalin notamment), en particulier selon le pH final imposé à la composition.
Les α-hydroxyacides (alpha hydroxyacide) sont par exemple choisis parmi l'acide citrique, l'acide lactique, l'acide méthyllactique, l'acide glucuronique, l'acide glycolique, l'acide pyruvique, l'acide 2-hydroxy-butanoïque, l'acide 2-hydroxypentanoïque, l'acide 2-hydroxyhexanoïque, l'acide 2-hydroxyheptanoïque, l'acide 2-hydroxyoctanoïque, l'acide 2-hydroxy-nonanoïque, l'acide 2-hydroxydécanoïque, l'acide 2-hydroxyundécanoïque, l'acide 2-hydroxydodécanoïque, l'acide 2-hydroxytétradécanoïque, l'acide 2-hydroxy-hexadécanoïque, l'acide 2-hydroxyoctadécanoïque, l'acide 2-hydroxytétra-cosanoïque, l'acide 2-hydroxyeïcosanoïque ; l'acide mandélique ; l'acide phényllactique ; l'acide gluconique ; l'acide galacturonique ; l'acide aleuritique ; l'acide ribonique ; l'acide tartronique ; l'acide tartrique ; l'acide malique ; l'acide fumarique ; leurs sels et leurs mélanges. On peut aussi utiliser des mélanges de ces différents acides.

Selon un mode préféré, l'alphahydroxyacide est choisi parmi l'acide citrique, l'acide malique, l'acide tartrique, l'acide lactique et leurs sels. Plus particulièrement, l'alphahydroxyacide est choisi parmi l'acide citrique, l'acide lactique, leurs sels et leurs mélanges.

Le ou les alphahydroxyacides peuvent être présents en une quantité allant par exemple de 0,001 à 10% en poids, de 0,005 à 5 % en poids, de préférence de 0,01 à 3 % et mieux de 0,02 à 1 % en poids par rapport au poids total de la composition.

De préférence, le rapport en poids alphahydroxyacide(s) / mono et/ou disaccharide(s) va de 0,01 à 20 et mieux de 0,05 à 10 et encore préférentiellement de 0,1 à 1.

De préférence, le rapport en poids alphahydroxyacide(s) / composé de type céramide(s) va de 1 à 200 et mieux de 1 à 20 et encore préférentiellement de 2 à 10.

De préférence, le rapport en poids mono et/ou disaccharide(s) / composé de type céramide(s) va de 0,1 à 100 et mieux de 1 à 50 et encore préférentiellement de 20 à 50.

Les compositions de l'invention contiennent en outre avantageusement au moins un agent tensioactif qui est généralement présent en une quantité comprise entre 0,1% et 50% en poids environ, de préférence entre 3% et 40% et encore plus préférentiellement entre 5% et 30%, par rapport au poids total de la composition.

Cet agent tensioactif peut être choisi parmi les agents tensioactifs anioniques, amphotères, non-ioniques, cationiques ou leurs mélanges.

Les tensioactifs convenant à la mise en oeuvre de la présente invention sont notamment les suivants :

### (i) Tensioactif(s) anionique(s) :

Leur nature ne revêt pas, dans le cadre de la présente invention, de caractère véritablement critique.
Ainsi, à titre d'exemple de tensioactifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés ,les acides alkyl(C₆-C₂₄) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersufates et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensioactifs non-ioniques rentrant particulièrement bien dans le cadre de la présente invention.

### (iii) Tensioactif(s) amphotère(s):

Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits commercialisés sous les dénomination MIRANOL, tels que décrits dans les brevets US-2 528 378 et US-2 781 354 et de structures :

R₂ -CONHCH₂CH₂ -N(R₃)(R₄)(CH₂COO-) (2)

dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₅-CONHCH₂CH₂-N(B)(C) (3)

dans laquelle :
B représente -CH₂CH₂OX' C représente -(CH₂)_{Z} -Y', avec z = 1 ou 2,
X' désigne le groupement -CH₂CH₂-COOH ou un atome d'hydrogène
Y' désigne -COOH ou le radical -CH₂ - CHOH - SO3H
R₅ désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL C2M concentré par la société RHONE POULENC.

On peut également utiliser des tensioactifs cationiques parmi lesquels on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkylammonium ou d'alkylpyridinium; les dérivés d'imidazoline ; ou les oxydes d'amines à caractère cationique.

La composition de l'invention peut également contenir au moins un additif choisi parmi les épaississants, les parfums, les agents nacrants, les conservateurs, les filtres solaires, les polymères anioniques ou non ioniques ou cationiques ou amphotères, les protéines, les hydrolysats de protéines, les acides gras à chaînes linéaires ou ramifiées en C₁₆-C₄₀ tels que l'acide méthyl-18 eicosanoique, les vitamines, le panthénol, les silicones volatiles ou non volatiles, organomodifiées ou non organomodifiées, les huiles végétales, animales, minérales ou synthétiques et tout autre additif classiquement utilisé dans le domaine cosmétique qui n'affecte pas les propriétés des compositions selon l'invention.

Les silicones préférées sont les silicones aminées et les polydiméthylsiloxanes, notamment les triméthylsilyl polydiméthylsiloxanes et les polydiméthylsiloxanes à groupements terminaux diméthylsilanol (dimethiconol).
Les polymères cationiques préférés sont les gommes de guar cationiques et les celluloses cationiques.

Ces additifs sont présents dans la composition selon l'invention dans des proportions pouvant aller de 0 à 50% en poids par rapport au poids total de la composition. La quantité précise de chaque additif est déterminée facilement par l'homme du métier selon sa nature et sa fonction.

Le milieu cosmétiquement acceptable est constitué uniquement par de l'eau ou par un mélange d'eau et d'au moins un solvant cosmétiquement acceptable tel qu'un alcool inférieur en C₁-C₄, tel que l'éthanol, l'isopropanol, le tertio-butanol, le n-butanol ; les alkylèneglycols comme le propylèneglycol, les éthers de polyols et leurs mélanges.
La composition comprend de 50 à 95 % en poids d'eau par rapport au poids total de la composition.

Le pH des compositions est généralement compris entre 2 et 12 et de préférence entre 4 et 9. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide minéral ou organique, de préférence un acide carboxylique tel que par exemple l'acide citrique.

Les compositions conformes à l'invention peuvent être plus particulièrement utilisées pour le lavage ou le traitement des matières kératiniques telles que les cheveux, la peau, les cils, les sourcils, les ongles, les lèvres, le cuir chevelu et plus particulièrement les cheveux.

En particulier, les compositions selon l'invention sont des compositions détergentes telles que des shampooings, des gels-douche et des bains moussants.

Dans ce mode de réalisation de l'invention, la quantité et la qualité des tensioactifs sont celles suffisantes pour conférer à la composition finale un pouvoir moussant et/ou détergent satisfaisant.

Ainsi, selon l'invention, les tensioactifs peuvent représenter de 4 % à 50 % en poids, de préférence de 6 % à 35 % en poids, et encore plus préférentiellement de 8 % à 25 % en poids, du poids total de la composition finale.
Le ou les tensioactifs peuvent être indifféremment choisis, seuls ou en mélanges, au sein des tensioactifs anioniques, amphotères, non ioniques tels que définis ci-dessus.

Dans les compositions conformes à l'invention, on utilise de préférence des mélanges d'agents tensioactifs et en particulier des mélanges d'agents tensioactifs anioniques et des mélanges d'agents tensioactifs anioniques et d'agents tensioactifs amphotères ou non ioniques. Un mélange particulièrement préféré est un mélange constitué d'au moins un agent tensioactif anionique et d'au moins un agent tensioactif amphotère.

On utilise de préférence un agent tensioactif anionique choisi parmi les alkyl(C₁₂-C₁₄) sulfates de sodium, de triéthanolamine ou d'ammonium, les alkyl (C₁₂-C₁₄)éthersulfates de sodium, de magnésium de triéthanolamine ou d'ammonium oxyéthylénés de 2 à 5 moles d'oxyde d'éthylène, le cocoyl iséthionate de sodium et l'alphaoléfine(C₁₄-C₁₆) sulfonate de sodium et leurs mélange avec :
- soit un agent tensioactif amphotère tel que les dérivés d'amine dénommés disodiumcocoamphodipropionate ou sodiumcocoamphopropionate commercialisés notamment par la société RHONE POULENC sous la dénomination commerciale "MIRANOL C2M CONC" en solution aqueuse à 38 % de matière active ou sous la dénomination MIRANOL C32;
- soit un agent tensioactif amphotère de type zwittérionique tel que les alkylbétaïnes en particulier la cocobétaïne commercialisée sous la dénomination "DEHYTON AB 30" en solution aqueuse à 32 % de MA par la société HENKEL ou les alkylamidobétaines en particulier les cocoamidopropylbetaines..

L'invention a encore pour objet un procédé de traitement des matières kératiniques telles que les cheveux, caractérisé en ce qu'il consiste à appliquer sur les matières kératiniques une composition cosmétique telle que définie précédemment, puis à effectuer éventuellement un rinçage à l'eau.

Ainsi, ce procédé selon l'invention permet le soin ou le lavage des cheveux ou de toute autre matière kératinique.

Les compositions de l'invention peuvent également se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une coloration, d'une permanente ou d'un défrisage.

Les compositions selon l'invention peuvent être utilisées comme produits non-rincés notamment pour le maintien de la coiffure, la mise en forme ou le coiffage des cheveux.

Elles sont alors plus particulièrement des lotions de mise en plis, des lotions pour le brushing, des compositions de fixation (laques) et de coiffage.

Les compositions de l'invention peuvent encore se présenter sous la forme de compositions lavantes pour la peau, et en particulier sous la forme de solutions ou de gels pour le bain ou la douche ou de produits démaquillants.

Les compositions selon l'invention peuvent également se présenter sous forme de lotions aqueuses ou hydroalcooliques pour le soin de la peau et/ou des cheveux.
La composition selon l'invention peut être utilisée pour le maquillage des matières kératiniques. La composition de maquillage peut être un vernis à ongles, une composition de soin des ongles, un eye-liner, un mascara, et de préférence un mascara.
Les compositions cosmétiques selon l'invention peuvent se présenter sous forme de gel, de lait, de crème, de lotion, de stick, d'émulsion, de lotion épaissie ou de mousse et être utilisées pour la peau, les ongles, les cils, les lèvres et plus particulièrement les cheveux et les cils.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des cheveux.

Lorsque la composition selon l'invention est conditionnée sous forme d'aérosol en vue d'obtenir une laque ou une mousse aérosol, elle comprend au moins un agent propulseur qui peut être choisi parmi les hydrocarbures volatils tels que le n-butane, le propane, l'isobutane, le pentane, un hydrocarbure chloré et/ou fluoré et leurs mélanges. On peut également utiliser en tant qu'agent propulseur le gaz carbonique, le protoxyde d'azote, le diméthyléther, l'azote, l'air comprimé et leurs mélanges.

Dans tout ce qui suit ou ce qui précède, les pourcentages exprimés sont en poids.

L'invention va être maintenant plus complètement illustrée à l'aide des exemples suivants qui ne sauraient être considérés comme la limitant aux modes de réalisation décrits.
Dans les exemples, MA signifie matière active.

### EXEMPLE 1

On a préparé la composition de shampooing suivante :

| | A | B |
|---|---|---|
| Lauryléther sulfate à 2,2 moles d'oxyde d'éthylène en solution aqueuse à 70% MA | 16 g MA | 16 g MA |
| Cocoyl bétaine à 30% MA | 1,8 g MA | 1,8 g MA |
| N-oléoyldihydrosphingosine | 0,01 g | - |
| Acide citrique | 0,05 g | 0,05 g |
| Acide lactique | 0,03 g | 0,03 g |
| Fructose | 0,25 g | 0,25 g |
| Glucose | 0,13 g | 0,13 g |
| Saccharose | 0,03 g | 0,03 g |
| Cellulose quaternisée (JR 400 de AMERCHOL) | 0,36 g | 0,36 g |
| Distéarate d'éthylèneglycol | 2 g | 2 g |
| Polydiméthylsiloxane (MIRASIL DM 500.000 de RHODIA) | 1 g | 1 g |
| Monoisopropanolamide d'acide de coprah | 1,13 g | 1,13 g |
| Glycolate de sodium | 0,12 g | 0,12 g |
| N-cocoyl amidoéthyl,Néthoxycarboxyméthyl glycinate de sodium | 0,62 gMA | 0,62 gMA |
| Conservateurs | qs | qs |
| Parfum | qs | qs |
| Eau qsp | 100 g | 100 g |

### Test de brushing :

On applique 1 g de composition sur des mèches de 2,7 g de cheveux préalablement lavés et essorés. On fait mousser la composition. On laisse pauser pendant 5 minutes, on rince les mêches à l'eau.

Sur les cheveux essorés, on réalise un brushing à l'aide d'une brosse et d'un sèche cheveux. Le brushing est réalisé en passant la brosse de la racine à la pointe. Les cheveux cassés sont récupérés minutieusement sur la brosse à l'aide d'un peigne, puis ils sont pesés. La masse de cheveux récupérés pour une mèche est ramenée à la masse de cheveux récupérés pour un gramme de cheveux de départ.

On a comparé la masse de cheveux récupérés pour les compositions A et B. Plus la masse de cheveux cassés est élevée, moins la composition protège les cheveux.

Les résultats sont rassemblés dans le tableaux ci-dessous :

| | | |
|---|---|---|
| Compositions testées | **A Invention** | **B** Comparatif |
| Masse des cheveux récupérés sur la brosse après brushing | **12,8 mg/g** | 21,6 mg/g |

On remarque que la masse de cheveux récupérés sur la brosse après le brushing est nettement diminuée (- 40%) pour la composition selon l'invention A contenant à la fois le céramide, le saccharide et l'alphahydroxyacide.

### EXEMPLES 2 ET 3

On peut préparer les compositions de shampooing suivantes :

| | 2 | 3 |
|---|---|---|
| Lauryléther sulfate à 2,2 moles d'oxyde d'éthylène en solution aqueuse à 70% MA | 14 g MA | 15,4 g MA |
| N-cocoylamidoéthyl N-éthoxycarboxy méthyl glycinate de sodium | 2,4 g MA | - |
| Cocoamidopropyl bétaine à 30% MA | - | 2,4 g MA |
| Chlorure de sodium | - | 0,75 g |
| N-oléoyldihydrosphingosine | 0,1 g | - |
| N-stéaroylphytosphingosine | | 0,05 g |
| Acide tartrique | 0,5 g | - |
| Acide malique | - | 0,2 g |
| Galactose | - | 1 g |
| Xylose | 0,1 g | - |
| Distéaryléther | - | 1,5 g |
| Mélange d'alcools gras en C18-C22 (NAFOL 1822C) | - | 1,5 g |
| Alcool laurique à 2,5 moles d'oxyde d'éthylène | - | 0,9 g |
| Chlorure d'hydroxypropylguar triméthylammonim | 0,2 g | 0,1 g |
| Distéarate d'éthylèneglycol | 2,5 g | - |
| Polydiméthylsiloxane (DOW CORNING 200 FLUID de DOW CORNING) | 2,5 g | 2,5 g |
| Monoisopropanolamide d'acide de coprah | 1,4 g | 1,5 g |
| Acide polyacrylique réticulé (CARBOPOL 980 deNOVEON) | 0,15 g | 0,2 g |
| Hexylèneglycol | 0,6 g | - |
| Conservateurs | qs | qs |
| Parfum | qs | qs |
| Eau qsp | 100 g | 100 g |

### EXEMPLES 4

On peut préparer la composition de mascara suivante :

| | | |
|---|---|---|
| Cire d'abeille | | 25% |
| Acide stéarique | | 5,82% |
| Triéthanolamine | | 2,4 % |
| Hydroxyethylcellulose | | 0,9% |
| Gomme arabique | | 3,4 % |
| Oxyde de fer noir | | 7% |
| N-oléoyldihydrosphingosine | | 0;1 % |
| Fructose | | 0,3 % |
| Acide citrique | | 0,5% |
| Conservateurs | | qs |
| Eau | qsp | 100 % |

## Revendications

1. Composition cosmétique à application topique, destinée au traitement des matières kératiniques, notamment des fibres kératiniques, en particulier les cheveux, **caractérisée par le fait qu'**elle comprend dans un milieu cosmétiquement acceptable,
au moins un monosaccharide et/ou un disaccharide présent(s) dans des concentrations allant de 0,05% à 10% en poids par rapport au poids total de la composition, au moins un alphahydroxyacide et au moins un composé de type céramide; la composition comprenant de 50 à 95% en poids d'eau par rapport au poids total de la composition.

2. Composition selon la revendication 1, **caractérisée par le fait que** le composé de type céramide répond à la formule générale (I): 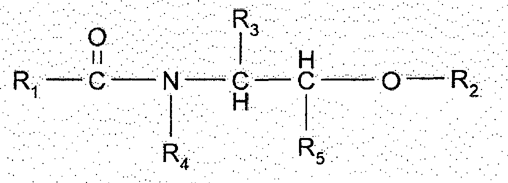 dans laquelle :
- R₁ désigne:
- soit un radical hydrocarboné, linéaire ou ramifié, saturé ou insaturé, en C₁-C₅₀, de préférence en C₅-C₅₀, ce radical pouvant être substitué par un ou plusieurs groupements hydroxyle éventuellement estérifié par un acide R₇COOH, R₇ étant un radical hydrocarboné, saturé ou insaturé, linéaire ou ramifié, éventuellement mono ou polyhydroxylé, en C₁-C₃₅, le ou les hydroxyles du radical R₇ pouvant être estérifié par un acide gras saturé ou insaturé, linéaire ou ramifié, éventuellement mono ou polyhydroxylé, en C₁-C₃₅;
- soit un radical R"-(NR-CO)-R', R désigne un atome d'hydrogène ou un radical hydrocarboné C₁-C₂₀ mono ou polyhydroxylé, préférentiellement monohydroxylé, R' et R" sont des radicaux hydrocarbonés dont la somme des atomes de carbone est comprise entre 9 et 30, R' étant un radical divalent.
- soit un radical R₈-O-CO-(CH2)ₚ, R₈ désigne un radical hydrocarboné en C₁-C₂₀, p est un entier variant de 1 à 12.
- R₂ est choisi parmi un atome d'hydrogène, un radical de type saccharidique, en particulier un radical (glycosyle)ₙ, (galactosyle)ₘ ou sulfogalactosyle, un résidu de sulfate ou de phosphate, un radical phosphoryléthylamine et un radical phosphoryléthylammonium, dans lesquels n est un entier variant de 1 à 4 et m est un entier variant de 1 à 8 ;
- R₃ désigne un atome d'hydrogène ou un radical hydrocarboné en C₁-C₃₃, saturé ou insaturé, hydroxylé ou non, le ou les hydroxyles pouvant être estérifés par un acide minéral ou un acide R₇COOH, R₇ ayant les mêmes significations que ci-dessus, le ou les hydroxyles pouvant être éthérifiés par un radical (glycosyle)ₙ, (galactosyle)ₘ, sulfogalactosyle, phosphoryléthylamine ou phosphoryléthylammonium, R₃ pouvant également être substitué par un ou plusieurs radicaux alkyle en C₁-C₁₄ ;
de préférence, R₃ désigne un radical α-hydroxyalkyle en C₁₅-C₂₆, le groupement hydroxyle étant éventuellement estérifié par un α-hydroxyacide en C₁₆-C₃₀ ;
- R₄ désigne un atome d'hydrogène, un radical méthyle, éthyle, un radical hydrocarboné en C₃-C₅₀, saturé ou insaturé, linéaire ou ramifié, éventuellement hydroxylé ou un radical -CH₂-CHOH-CH₂-O-R₆ dans lequel R₆ désigne un radical hydrocarboné en C₁₀-C₂₆ ou un radical R₈-O-CO-(CH₂)ₚ, R₈ désigne un radical hydrocarboné en C₁-C₂₀, p est un entier variant de 1 à 12,
- R₅ désigne un atome d'hydrogène ou un radical hydrocarboné en C₁-C₃₀ saturé ou insaturé, linéaire ou ramifié, éventuellement mono ou polyhydroxylé, le ou les hydroxyles pouvant être éthérifiés par un radical (glycosyle)ₙ, (galactosyle)ₘ, sulfogalactosyle, phosphoryléthylamine ou phosphoryléthylammonium,
sous réserve que lorsque R₃ et R₅ désignent hydrogène ou lorsque R₃ désigne hydrogène et R₅ désigne méthyle alors R₄ ne désigne pas un atome d'hydrogène, un radical méthyle ou éthyle.

3. Composition selon l'une quelconque des revendications précédentes, **caractérisé en ce que** le composé de type céramide est choisi dans le groupe constitué par :
- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1 ,3-diol,
- le 2-N-béhénoylamino-octadécane-1,3-dioi,
- le 2-N-[2-hydroxy-palmitoyl]-amino-octadécane-1,3-diol,
- le 2-N-stéaroyl amino-octadécane-1,3,4 triol,
- le 2-N-palmitoylamino-hexadécane-1,3-diol,
ou les mélanges de ces composés.

4. Composition selon l'une quelconque des revendications 1 ou 2, **caractérisé en ce que** le composé de type céramide est choisi parmi le bis-(N-hydroxyéthyl N-cétyl) malonamide, le N-(2-hydroxyéthyl)-N-(3-cétyloxy-2-hydroxypropyl)amide d'acide cétylique) et le N-docosanoyl N-méthyl-D-glucamine.

5. Composition selon l'une quelconque des revendications précédentes, **caractérisée par le fait que** le ou les composés de type céramides sont présents dans des concentrations allant de 0,0001% à 10% en poids environ par rapport au poids total de la composition, et de préférence de 0,001 à 5% environ et encore plus préférentiellement de 0,005 à 1 % en poids

6. Composition selon l'une quelconque des revendications précédentes, **caractérisée par le fait que** le monosaccharide ou le disaccharide est choisi parmi le glucose, le fructose, le galactose, le mannose, le talose, le sorbose, le xylose, le lyxose, le fucose, le fucose, le arabinose, le rhamnose, le ribose, le ribulose, le xylulose, le maltose, le lactose, le cellobiose, le tréhalose, le saccharose et leurs mélanges.

7. Composition selon l'une quelconque des revendications précédentes, **caractérisée par le fait que** le monosaccharide est choisi parmi le D-glucose, le D-fructose, le saccharose et leurs mélanges

8. Composition selon l'une quelconque des revendications précédentes, **caractérisée par le fait que** ledit mono ou di saccharide est présent dans des concentrations allant de 0,1 à 5% en poids environ par rapport au poids total de la composition, préférentiellement de 0,1 à 1% en poids.

9. Composition selon l'une quelconque des revendications précédentes, **caractérisée par le fait que** ledit alphahydroxyacide est choisi parmi l'acide citrique, l'acide lactique, l'acide méthyllactique, l'acide glucuronique, l'acide glycolique, l'acide pyruvique, l'acide 2-hydroxy-butanoïque, l'acide 2-hydroxypentanoïque, l'acide 2-hydroxyhexanoïque, l'acide 2-hydroxyheptanoïque, l'acide 2-hydroxyoctanoïque, l'acide 2-hydroxy-nonanoïque, l'acide 2-hydroxydécanoïque, l'acide 2-hydroxyundécanoïque, l'acide 2-hydroxydodëcanoïque, l'acide 2-hydroxytétradécanoïque, l'acide 2-hydroxy-hexadécanoïque, l'acide 2-hydroxyoctadécanoïque, l'acide 2-hydroxytétra-cosanoïque, l'acide 2-hydroxyeïcosanoïque ; l'acide mandélique ; l'acide phényllactique ; l'acide gluconique ; l'acide galacturonique ; l'acide aleuritique ; l'acide ribonique ; l'acide tartronique ; l'acide tartrique ; l'acide malique ; l'acide fumarique ; leurs sels et leurs mélanges.

10. Composition selon la revendication précédente, **caractérisée par le fait que** ledit alphahydroxyacide est choisi parmi l'acide citrique, l'acide malique, l'acide tartrique, l'acide lactique, de préférence parmi l'acide citrique, l'acide lactique, leurs sels et leurs mélanges.

11. Composition selon l'une quelconque des revendications précédentes, **caractérisée par le fait que** ledit alphahydroxyacide est présent dans des concentrations allant de 0,001 à 10% en poids par rapport au poids total de la composition et de préférence de 0,005 à 5 % en poids et plus préférentiellement de 0,01 à 3% en poids.

12. Composition selon l'une quelconque des revendications précédentes, **caractérisée par le fait que** le rapport en poids alphahydroxyacide(s) / mono et/ou disaccharide(s) va de 0,01 à 20 et mieux de 0,05 à 10.

13. Composition selon l'une quelconque des revendications précédentes, **caractérisée par le fait que** le rapport en poids alphahydroxyacide(s) / composé de type céramide(s) va de 1 à 200 et mieux de 1 à 20.

14. Composition selon l'une quelconque des revendications précédentes, **caractérisée par le fait que** le rapport en poids mono et/ou disaccharide(s) / composé de type céramide(s) va de 0,1 à 100 et mieux de 1 à 50.

15. Composition selon l'une quelconque des revendications précédentes, **caractérisée par le fait qu'**elle comprend en outre au moins un agent tensioactif choisi parmi les tensioactifs anioniques, cationiques, non ioniques, amphotères et leurs mélanges.

16. Composition selon la revendication 15, **caractérisée par le fait que** le ou les agents tensioactifs sont présents à une concentration comprise entre 0,1% et 60% en poids, de préférence entre 3% et 40% en poids, et encore plus préférentiellement entre 5% et 30% en poids, par rapport au poids total de la composition.

17. Composition selon l'une quelconque des revendications 1 à 16, **caractérisée par le fait qu'**elle comprend en outre au moins un additif choisi parmi les épaississants, les parfums, les agents nacrants, les conservateurs, les filtres solaires, les polymères anioniques ou non ioniques ou cationiques ou amphotères, les protéines, les hydrolysats de protéines, les acides gras à chaînes linéaires ou ramifiées en C₁₆-C₄₀ tels que l'acide méthyl-18 eicosanoique, les silicones volatiles ou non volatiles, organomodifiées ou non organomodifiées, les vitamines, le panthénol, et les esters gras et leurs mélanges.

18. Composition selon l'une quelconque des revendications précédentes, **caractérisée par le fait qu'**elle comprend en outre au moins un polymère cationique.

19. Composition selon l'une quelconque des revendications précédentes, **caractérisée par le fait qu'**elle comprend en outre au moins une silicone.

20. Composition selon l'une quelconque des revendications précédentes, **caractérisée par le fait que** le milieu cosmétiquement acceptable est constitué par de l'eau ou un mélange d'eau et d'au moins un solvant cosmétiquement acceptable.

21. Composition selon la revendication 20, **caractérisée par le fait que** les solvants cosmétiquement acceptables sont choisis dans le groupe constitué par les monoalcools, les polyalcools, les éthers de polyols, et leurs mélanges.

22. Composition selon l'une quelconque des revendications 1 à 21, **caractérisées par le fait qu'**elle se présente sous forme de shampooing, d'après-shampooing, de composition pour la permanente, le défrisage, la coloration ou la décoloration des cheveux, de composition à rincer à appliquer entre les deux étapes d'une coloration, d'une permanente ou d'un défrisage, de composition lavantes pour la peau.

23. Utilisation d'une composition telle que définie selon l'une quelconque des revendications 1 à 22 pour protéger les cheveux pendant les brushings.

24. Utilisation d'une composition telle que définie selon l'une quelconque des revendications 1 à 22 pour diminuer la masse de cheveux recueillie pendant les brushings.

## Patentansprüche

1. Kosmetische Zusammensetzung zur topischen Anwendung, die für die Behandlung von Keratinmaterial, insbesondere Keratinfasern, speziell des Haars, bestimmt ist, **dadurch gekennzeichnet, dass** sie mindestens ein Monosaccharid und/oder Disaccharid, das/die in Konzentrationen von 0,05 Gew.-% bis 10 Gew.-% in Bezug auf das Gesamtgewicht der Zusammensetzung vorliegt/vorliegen, mindestens eine alpha-Hydroxysäure und mindestens eine ceramidartige Verbindung in einem kosmetisch unbedenklichen Medium umfasst; wobei die Zusammensetzung 50 bis 95 Gew.-% Wasser in Bezug auf das Gesamtgewicht der Zusammensetzung umfasst.

2. Zusammensetzung nach Anspruch 1, **dadurch gekennzeichnet, dass** die ceramidartige Verbindung die allgemeine Formel (I) aufweist: 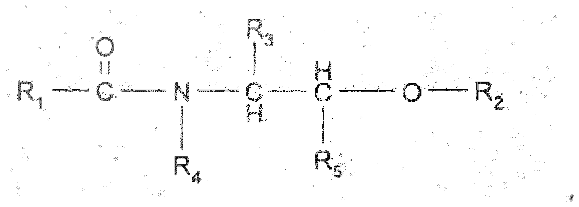 in der
- R₁ Folgendes bedeutet:
- entweder einen geradkettigen oder verzweigten, gesättigten oder ungesättigten C₁-C₅₀-Kohlenwasserstoffrest, vorzugsweise C₅-C₅₀-Kohlenwasserstoffrest, wobei dieser Rest durch eine oder mehrere Hydroxylgruppen substituiert sein kann, die gegebenenfalls mit einer Säure R₇COOH verestert sein können, wobei R₇ einen gesättigten oder ungesättigten, geradkettigen oder verzweigten, gegebenenfalls mono- oder polyhydroxylierten C₁-C₃₅-Kohlenwasserstoffrest bedeutet, wobei die Hydroxylgruppe(n) des Rests R₇ mit einer gesättigten oder ungesättigten, geradkettigen oder verzweigten, gegebenenfalls mono- oder polyhydroxylierten C₁-C₃₅-Fettsäure verestert sein kann/können;
- oder einen Rest R"-(NR-CO)-R', wobei R ein Wasserstoffatom oder einen mono- oder polyhydroxylierten, vorzugsweise monohydroxylierten, C₁-C₂₀-Kohlenwasserstoffrest bedeutet, R' und R" Kohlenwasserstoffreste bedeuten, bei denen die Summe der Kohlenstoffatome zwischen 9 und 30 beträgt, wobei R' einen zweiwertigen Rest bedeutet;
- oder einen Rest R₈-O-CO-(CH₂)ₚ, wobei R₈ einen C₁-C₂₀-Kohlenwasserstoffrest bedeutet und p eine ganze Zahl von 1 bis 12 bedeutet;
- R₂ aus einem Wasserstoffatom, einem saccharidartigen Rest, insbesondere einem (Glycosyl)ₙ-, (Galactosyl)ₘ- oder Sulfogalactosylrest, einem Sulfat- oder Phosphatrest, einem Phosphorylethylaminrest und einem Phosphorylethylammoniumrest, in denen n eine ganze Zahl von 1 bis 4 bedeutet und m eine ganze Zahl von 1 bis 8 bedeutet, ausgewählt ist;
- R₃ ein Wasserstoffatom oder einen gesättigten oder ungesättigten, gegebenenfalls hydroxylierten C₁-C₃₃-Kohlenwasserstoffrest, wobei die Hydroxylgruppe(n) mit einer Mineralsäure oder einer Säure R₇COOH verestert sein kann/können, wobei R₇ die gleichen Bedeutungen wie oben aufweist, wobei der oder die Hydroxylreste mit einem (Glycosyl)ₙ-, (Galactosyl)ₘ-, Sulphogalactosyl-, Phosphorylethylamin- oder Phosphorylethylammoniumrest verethert sein können, wobei R₃ auch durch einen oder mehrere C₁-C₁₄-Alkylreste substituiert sein kann, bedeutet;
vorzugsweise bedeutet R₃ einen C₁₅-C₂₆-α-Hydroxyalkylrest, wobei die Hydroxylgruppe gegebenenfalls mit einer C₁₆-C₃₀-α-Hydroxysäure verestert ist;
- R₄ ein Wasserstoffatom, einen Methylrest, einen Ethylrest, einen gesättigten oder ungesättigten, geradkettigen oder verzweigten, gegebenenfalls hydroxylierten C₃-C₅₀-Kohlenwasserstoffrest oder einen Rest -CH₂-CHOH-CH₂-O-R₆, wobei R₆ einen C₁₀-C₂₆-Kohlenwasserstoffrest bedeutet, oder einen Rest R₈-O-CO-(CH₂)ₚ, wobei R₈ einen C₁-C₂₀-Kohlenwasserstoffrest bedeutet und p eine ganze Zahl von 1 bis 12 bedeutet, bedeutet;
- R₅ ein Wasserstoffatom oder einen gesättigten oder ungesättigten, geradkettigen oder verzweigten, gegebenenfalls mono- oder polyhydroxylierten C₁-C₃₀-Kohlenwasserstoffrest bedeutet, wobei die Hydroxylgruppe(n) durch einen (Glycosyl)ₙ-, (Galactosyl)ₘ-, Sulphogalactosyl-, Phosphorylethylamin- oder Phosphorylethylammoniumrest verethert sein können, bedeutet,
mit der Maßgabe, dass, wenn R₃ und R₅ Wasserstoff bedeuten oder wenn R₃ Wasserstoff und R₅ Methyl bedeuten, dann R₄ nicht ein Wasserstoffatom, einen Methylrest oder einen Ethylrest bedeutet.

3. Zusammensetzung nach einem der vorhergehenden Ansprüche, **dadurch gekennzeichnet, dass** die ceramidartige Zusammensetzung aus der Gruppe bestehend aus den Folgenden ausgewählt ist:
- 2-N-Linoleoylaminooctadecan-1,3-diol,
- 2-N-Oleoylaminooctadecan-1,3-diol,
- 2-N-Palmitoylaminooctadecan-1,3-diol,
- 2-N-Stearoylaminooctadecan-1,3-diol,
- 2-N-Behenoylaminooctadecan-1,3-diol,
- 2-N-[2-Hydroxypalmitoyl]aminooctadecan-1,3-diol,
- 2-N-Stearoylaminooctadecan-1,3,4-triol,
- 2-N-Palmitoylaminohexadecan-1,3-diol
oder den Mischungen dieser Verbindungen.

4. Zusammensetzung nach einem der Ansprüche 1 oder 2, **dadurch gekennzeichnet, dass** die ceramidartige Verbindung aus Bis(N-hydroxyethyl-N-cetyl)malonamid, Cetylsäure-N-(2-Hydroxyethyl)-N-(3-cetyloxy-2-hydroxypropyl)amid und N-Docosanoyl-N-methyl-D-glucamin ausgewählt ist.

5. Zusammensetzung nach einem der vorhergehenden Ansprüche, **dadurch gekennzeichnet, dass** die ceramidartige(n) Verbindung(en) in Konzentrationen von 0,0001 Gew.-% bis 10 Gew.-% (ungefähr), vorzugsweise 0,001 bis 5% und noch stärker bevorzugt von 0,005 bis 1 Gew.-% in Bezug auf das Gesamtgewicht der Zusammensetzung vorliegen.

6. Zusammensetzung nach einem der vorhergehenden Ansprüche, **dadurch gekennzeichnet, dass** das Monosaccharid oder Disaccharid aus Glukose, Fruktose, Galaktose, Mannose, Talose, Sorbose, Xylose, Lyxose, Fucose, Arabinose, Rhamnose, Ribose, Ribulose, Xylulose, Maltose, Lactose, Cellobiose, Trehalose, Saccharose und ihren Mischungen ausgewählt ist.

7. Zusammensetzung nach einem der vorhergehenden Ansprüche, **dadurch gekennzeichnet, dass** das Monosaccharid aus D-Glukose, D-Fruktose, Saccharose und ihren Mischungen ausgewählt ist.

8. Zusammensetzung nach einem der vorhergehenden Ansprüche, **dadurch gekennzeichnet, dass** das Mono- oder Disaccharid in Konzentrationen von 0,1 bis 5 Gew.-% (ungefähr), vorzugsweise 0,1 bis 1 Gew.-%, in Bezug auf das Gesamtgewicht der Zusammensetzung vorliegt.

9. Zusammensetzung nach einem der vorhergehenden Ansprüche, **dadurch gekennzeichnet, dass** die alpha-Hydroxysäure aus Citronensäure, Milchsäure, Methylmilchsäure, Glucuronsäure, Glycolsäure, Brenztraubensäure, 2-Hydroxybutansäure, 2-Hydroxypentansäure, 2-Hydroxyhexansäure, 2-Hydroxyheptansäure, 2-Hydroxyoctansäure, 2-Hydroxynonansäure, 2-Hydroxydecansäure, 2-Hydroxyundecansäure, 2-Hydroxydodecansäure, 2-Hydroxytetradecansäure, 2-Hydroxyhexadecansäure, 2-Hydroxyoctadecansäure, 2-Hydroxytetracosansäure, 2-Hydroxyeicosansäure, Mandelsäure, Phenylmilchsäure, Gluconsäure, Galacturonsäure, Aleuritinsäure, Ribonsäure, Tartronsäure, Weinsäure, Äpfelsäure, Fumarsäure, ihren Salzen und ihren Mischungen.

10. Zusammensetzung nach dem vorhergehenden Anspruch, **dadurch gekennzeichnet, dass** die alpha-Hydroxysäure aus Citronensäure, Äpfelsäure, Weinsäure, Milchsäure, vorzugsweise aus Citronensäure, Milchsäure, ihren Salzen und ihren Mischungen ausgewählt ist.

11. Zusammensetzung nach einem der vorhergehenden Ansprüche, **dadurch gekennzeichnet, dass** die alpha-Hydroxysäure in Konzentrationen von 0,001 bis 10 Gew.-%, vorzugsweise 0,005 bis 5 Gew.-% und stärker bevorzugt 0,01 bis 3 Gew.-% in Bezug auf das Gesamtgewicht der Zusammensetzung vorliegt.

12. Zusammensetzung nach einem der vorhergehenden Ansprüche, **dadurch gekennzeichnet, dass** das Gewichtsverhältnis von alpha-Hydroxysäure(n) zu Mono- und/oder Disaccharid(en) bei 0,01 zu 20, besser 0,05 zu 10, liegt.

13. Zusammensetzung nach einem der vorhergehenden Ansprüche, **dadurch gekennzeichnet, dass** das Gewichtsverhältnis von alpha-Hydroxysäure(n) zu ceramidartige(n) Verbindung(en) bei 1 zu 200, besser 1 zu 20, liegt.

14. Zusammensetzung nach einem der vorhergehenden Ansprüche, **dadurch gekennzeichnet, dass** das Gewichtsverhältnis von Mono- und/oder Disaccharid(en) zu ceramidartige(n) Verbindung(en) bei 0,1 zu 100, besser 1 zu 50, liegt.

15. Zusammensetzung nach einem der vorhergehenden Ansprüche, **dadurch gekennzeichnet, dass** sie weiterhin mindestens ein Tensid, ausgewählt aus anionischen, kationischen, nichtionischen, amphoteren Tensiden und ihren Mischungen, umfasst.

16. Zusammensetzung nach Anspruch 15, **dadurch gekennzeichnet, dass** das Tensid/die Tenside in einer Konzentration zwischen 0,1 Gew.-% und 60 Gew.-%, vorzugsweise zwischen 3 Gew.-% und 40 Gew.-%, noch stärker bevorzugt zwischen 5 Gew.-% und 30 Gew.-% in Bezug auf das Gesamtgewicht der Zusammensetzung vorliegen.

17. Zusammensetzung nach einem der Ansprüche 1 bis 16, **dadurch gekennzeichnet, dass** sie weiterhin mindestens einen Zusatzstoff, ausgewählt aus Verdickungsmitteln, Duftstoffen, Perlglanzmitteln, Konservierungsmitteln, Sonnenschutzfiltern, anionischen oder nichtionischen oder kationischen oder amphoteren Polymeren, Proteinen, Proteinhydrolysaten, geradkettigen oder verzweigten C₁₆-C₄₀-Fettsäuren wie Methyl-18-ecosansäure, flüchtigen oder nichtflüchtigen, gegebenenfalls organisch modifizierten Silikonen, Vitaminen, Panthenol, Fettsäureestern und ihren Mischungen umfasst.

18. Zusammensetzung nach einem der vorhergehenden Ansprüche, **dadurch gekennzeichnet, dass** sie weiterhin mindestens ein kationisches Polymer umfasst.

19. Zusammensetzung nach einem der vorhergehenden Ansprüche, **dadurch gekennzeichnet, dass** sie weiterhin mindestens ein Silikon umfasst.

20. Zusammensetzung nach einem der vorhergehenden Ansprüche, **dadurch gekennzeichnet, dass** das kosmetisch unbedenkliche Medium aus Wasser oder einer Mischung aus Wasser und mindestens einem kosmetisch unbedenklichen Lösungsmittel besteht.

21. Zusammensetzung nach Anspruch 20, **dadurch gekennzeichnet, dass** die kosmetisch unbedenklichen Lösungsmittel aus der Gruppe bestehend aus Monoalkoholen, Polyalkoholen, Polyolethern und ihren Mischungen ausgewählt sind.

22. Zusammensetzung nach einem der Ansprüche 1 bis 21, **dadurch gekennzeichnet, dass** sie in Form eines Shampoos, eines Conditioners, einer Dauerwellzusammensetzung, einer Entkräuselungszusammensetzung, einer Färbezusammensetzung oder einer Entfärbezusammensetzung für das Haar, einer Spülzusammensetzung zum Einsatz zwischen den zwei Stufen einer Färbung, einer Dauerwelle oder einer Entkräuselung oder einer Waschzusammensetzung für die Haut vorliegt.

23. Verwendung einer wie oben in einem der Ansprüche 1 bis 22 definierten Zusammensetzung zum Schützen des Haars während des Haarföhnens.

24. Verwendung einer wie oben in einem der Ansprüche 1 bis 22 definierten Zusammensetzung zum Reduzieren des aufgenommenen Haars während des Haarföhnens.

## Claims

1. Cosmetic composition for topical application intended for the treatment of keratinous substances, in particular keratinous fibres, especially the hair, **characterized in that** it comprises, in a cosmetically acceptable medium, at least one monosaccharide and/or one disaccharide which is or are present in concentrations ranging from 0.05% to 10% by weight with respect to the total weight of the composition, at least one α-hydroxy acid and at least one compound of ceramide type; the composition comprising from 50 to 95% by weight of water, with respect to the total weight of the composition.

2. Composition according to Claim 1, **characterized in that** the compound of ceramide type corresponds to the general formula (I): 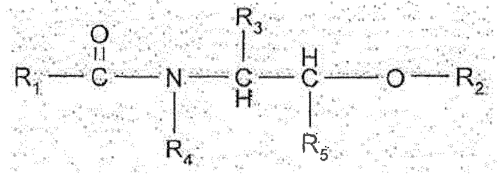 in which:
- R₁ denotes:
- either a saturated or unsaturated and linear or branched C₁-C₅₀, preferably C₅-C₅₀, hydrocarbon radical, it being possible for this radical to be substituted by one or more hydroxyl groups optionally esterified by an acid R₇COOH, R₇ being an optionally mono- or polyhydroxylated, saturated or unsaturated and linear or branched C₁-C₃₅ hydrocarbon radical, it being possible for the hydroxyl or hydroxyls of the R₇ radical to be esterified by an optionally mono- or polyhydroxylated, saturated or unsaturated and linear or branched C₁-C₃₅ fatty acid;
- or an R"-(NR-CO)-R' radical, in which R denotes a hydrogen atom or a mono- or polyhydroxylated, preferably monohydroxylated, C₁-C₂₀ hydrocarbon radical, R' and R'' are hydrocarbon radicals, the sum of the carbon atoms of which is between 9 and 30, R' being a divalent radical;
- or an R₈-O-CO-(CH₂)ₚ radical, in which R₈ denotes a C₁-C₂₀ hydrocarbon radical and p is an integer varying from 1 to 12;
- R₂ is chosen from a hydrogen atom, a radical of saccharide type, in particular a (glycosyl)ₙ, (galactosyl)ₘ or sulphogalactosyl radical, a sulphate or phosphate residue, a phosphorylethylamine radical and a phosphorylethylammonium radical, in which n is an integer varying from 1 to 4 and m is an integer varying from 1 to 8;
- R₃ denotes a hydrogen atom or a hydroxylated or nonhydroxylated and saturated or unsaturated C₁-C₃₃ hydrocarbon radical, it being possible for the hydroxyl or hydroxyls to be esterified by an inorganic acid or an acid R₇COOH, R₇ having the same meanings as hereinabove, and it being possible for the hydroxyl or hydroxyls to be etherified by a (glycosyl)ₙ, (galactosyl)ₘ, sulphogalactosyl, phosphorylethylamine or phosphorylethylammonium radical, it also being possible for R₃ to be substituted by one or more C₁-C₁₄ alkyl radicals;
preferably, R₃ denotes a C₁₅-C₂₆ α-hydroxyalkyl radical, the hydroxyl group optionally being esterified by a C₁₆-C₃₀ α-hydroxy acid;
- R₄ denotes a hydrogen atom, a methyl or ethyl radical, an optionally hydroxylated, saturated or unsaturated and linear or branched C₃-C₅₀ hydrocarbon radical or a - CH₂-CHOH-CH₂-O-R₆ radical, in which R₆ denotes a C₁₀-C₂₆ hydrocarbon radical, or an R₈-O-CO-(CH₂)ₚ radical, in which R₈ denotes a C₁-C₂₀ hydrocarbon radical and p is an integer varying from 1 to 12;
- R₅ denotes a hydrogen atom or an optionally mono- or polyhydroxylated, saturated or unsaturated and linear or branched C₁-C₃₀ hydrocarbon radical, it being possible for the hydroxyl or hydroxyls to be etherified by a (glycosyl)ₙ, (galactosyl)ₘ, sulphogalactosyl, phosphorylethylamine or phosphorylethylammonium radical;
with the proviso that, when R₃ and R₅ denote hydrogen or when R₃ denotes hydrogen and R₅ denotes methyl, then R₄ does not denote a hydrogen atom or a methyl or ethyl radical.

3. Composition according to either one of the preceding claims, **characterized in that** the compound of ceramide type is chosen from the group consisting of:
- 2-N-linoleoylaminooctadecane-1,3-diol,
- 2-N-oleoylaminooctadecane-1,3-diol,
- 2-N-palmitoylaminooctadecane-1,3-diol,
- 2-N-stearoylaminooctadecane-1,3-diol,
- 2-N-behenoylaminooctadecane-1,3-diol,
- 2-N-[2-hydroxypalmitoyl]aminooctadecane-1,3-diol,
- 2-N-stearoylaminooctadecane-1,3,4-triol,
- 2-N-palmitoylaminohexadecane-1,3-diol,
or the mixtures of these compounds.

4. Composition according to either one of Claims 1 and 2, **characterized in that** the compound of ceramide type is chosen from bis(N-hydroxyethyl-N-cetyl)-malonamide, the N-(2-hydroxyethyl)-N-(3-cetyloxy-2-hydroxypropyl)amide of cetylic acid and N-docosanoyl-N-methyl-D-glucamine.

5. Composition according to any one of the preceding claims, **characterized in that** the compound or compounds of ceramide type are present in concentrations ranging from 0.0001% to 10% by weight approximately with respect to the total weight of the composition, preferably from 0.001 to 5% approximately and more preferably still from 0.005 to 1% by weight.

6. Composition according to any one of the preceding claims, **characterized in that** the monosaccharide or the disaccharide is chosen from glucose, fructose, galactose, mannose, talose, sorbose, xylose, lyxose, fucose, arabinose, rhamnose, ribose, ribulose, xylulose, maltose, lactose, cellobiose, trehalose, sucrose and their mixtures.

7. Composition according to any one of the preceding claims, **characterized in that** the monosaccharide is chosen from D-glucose, D-fructose, sucrose and their mixtures.

8. Composition according to any one of the preceding claims, **characterized in that** the said mono- or disaccharide is present in concentrations ranging from 0.1 to 5% by weight approximately with respect to the total weight of the composition, preferably from 0.1 to 1% by weight.

9. Composition according to any one of the preceding claims, **characterized in that** the said α-hydroxy acid is chosen from citric acid, lactic acid, methyllactic acid, glucuronic acid, glycolic acid, pyruvic acid, 2-hydroxybutanoic acid, 2-hydroxypentanoic acid, 2-hydroxyhexanoic acid, 2-hydroxyheptanoic acid, 2-hydroxyoctanoic acid, 2-hydroxynonanoic acid, 2-hydroxydecanoic acid, 2-hydroxyundecanoic acid, 2-hydroxydodecanoic acid, 2-hydroxytetradecanoic acid, 2-hydroxyhexadecanoic acid, 2-hydroxyoctadecanoic acid, 2-hydroxytetracosanoic acid, 2-hydroxyeicosanoic acid, mandelic acid, phenyllactic acid, gluconic acid, galacturonic acid, aleuritic acid, ribonic acid, tartronic acid, tartaric acid, malic acid, fumaric acid, their salts and their mixtures.

10. Composition according to the preceding claim, **characterized in that** the said α-hydroxy acid is chosen from citric acid, malic acid, tartaric acid or lactic acid, preferably from citric acid, lactic acid, their salts and their mixtures.

11. Composition according to any one of the preceding claims, **characterized in that** the said α-hydroxy acid is present in concentrations ranging from 0.001 to 10% by weight with respect to the total weight of the composition, preferably from 0.005 to 5% by weight and more preferably from 0.01 to 3% by weight.

12. Composition according to any one of the preceding claims, **characterized in that** the α-hydroxy acid(s)/mono- and/or disaccharide(s) ratio by weight ranges from 0.01 to 20 and better still from 0.05 to 10.

13. Composition according to any one of the preceding claims, **characterized in that** the α-hydroxy acid(s)/compound of ceramide type ratio by weight ranges from 1 to 200 and better still from 1 to 20.

14. Composition according to any one of the preceding claims, **characterized in that** the mono- and/or disaccharide(s)/compound of ceramide type ratio by weight ranges from 0.1 to 100 and better still from 1 to 50.

15. Composition according to any one of the preceding claims, **characterized in that** it additionally comprises at least one surface-active agent chosen from anionic, cationic, nonionic or amphoteric surfactants and their mixtures.

16. Composition according to Claim 15, **characterized in that** the surface-active agent or agents are present at a concentration of between 0.1% and 60% by weight, preferably between 3% and 40% by weight and more preferably still between 5% and 30% by weight, with respect to the total weight of the composition.

17. Composition according to any one of Claims 1 to 16, **characterized in that** it additionally comprises at least one additive chosen from thickeners, fragrances, pearlescent agents, preservatives, sunscreens, anionic or nonionic or cationic or amphoteric polymers, proteins, protein hydrolysates, fatty acids with linear or branched C₁₆-C₄₀ chains, such as 18-methyleicosanoic acid, volatile or nonvolatile and organomodified or nonorganomodified silicones, vitamins, panthenol, fatty esters and their mixtures.

18. Composition according to any one of the preceding claims, **characterized in that** it additionally comprises at least one cationic polymer.

19. Composition according to any one of the preceding claims, **characterized in that** it additionally comprises at least one silicone.

20. Composition according to any one of the preceding claims, **characterized in that** the cosmetically acceptable medium is composed of water or a mixture of water and of at least one cosmetically acceptable solvent.

21. Composition according to Claim 20, **characterized in that** the cosmetically acceptable solvents are chosen from the group consisting of monoalcohols, polyalcohols, polyol ethers and their mixtures.

22. Composition according to any one of Claims 1 to 21, **characterized in that** it is provided in the form of a shampoo, conditioner, composition for perming, straightening, dyeing or bleaching the hair, rinse-out composition to be applied between the two stages of a dyeing, perming or hair-straightening operation, or washing composition for the skin.

23. Use of a composition as defined according to any one of Claims 1 to 22 for protecting the hair during blow-drying operations.

24. Use of a composition as defined according to any one of Claims 1 to 22 to reduce the weight of hair collected during blow-drying operations.
